# EUROPÄISCHE PATENTANMELDUNG

(11) **EP 1 233 062 A2**
(43) Veröffentlichungstag der Anmeldung: **21.08.2002**
(21) Anmeldenummer: 02001327.2
(22) Anmeldetag: 02.07.1993
(51) Int. Cl.: C12N 15/00, C12N 7/00, C12N 15/11

(54) **Instabilisierung von viralen Quasi-Spezies-Verteilungen unter Vermeidung von Resistenzphänomenen**

(30) Priorität: 07.07.1992 DE 4222289
(62) Teilanmeldung aus: 93915776.4
(71) Anmelder: Evotec OAI AG, 22525 Hamburg (DE)
(72) Erfinder: Eigen, Manfred, 37075 Göttingen (DE); Schwienhorst, Andreas, 37083 Göttingen (DE); Bierbricher, Christof, c/oDiagen Inst.molek.Diagn., 40724 Hilden (DE); Lindemann, Björn, 22397 Hamburg (DE); Domingo, Esteban, c/oDiagen Instit. molekul.Diagn., 40724 Hilden (DE); Holland, John, c/oDiagen Inst. molekular.Diagnost., 40724 Hilden (DE); Henco, Karsten, 40699 Erkrath (DE)
(74) Vertreter: Meyers, Hans-Wilhelm, Dr.Dipl.-Chem.

(57) **Zusammenfassung**

Verfahren zur Instabilisierung von viralen Quasi-Spezies-Verteilungen unter Vermeidung von Resistenzphänomenen durch Replikation der Nukleinsäuren der in der Quasi-Spezies-Vertellung vorhandenen Viren mittels eines fehlerhaften Replikationssystems,
- wobei das fehlerhafte Replikationssystem eine Fehleinbaurate für Nukleotide oberhalb der Fehleinbaurate des viralen Wild-Typ-Replikationssystem aufweist und
- wobei die Viren von dem Replikationssystem mit höherer Fehleinbaurate mindestens so effektiv repliziert werden wie es das Replikationssystem des Virus-Wildtyps leistet.

## Beschreibung

Die Erfindung betrifft ein Verfahren zur Instabilisierung von viralen Quasi-Spezies-Verteilungen unter Vermeidung von Resistenzphänomenen durch Replikation der Nukleinsäuren der in der Quasi-Spezies-Verteilung vorhandenen Viren mittels eines fehlerhaften Replikationssystems. Gegenstand der vorliegenden Erfindung ist auch ein Mittel zur Durchführung des erfindungsgemäßen Verfahrens sowie Nukleinsäuresequenzen erhältlich durch Reaktion von viralen Replikationssystemen mit Nukleotiden.

Viruserkrankungen haben sich in der bisherigen Pharmakologie als äußerst schwer therapierbar herausgestellt. Bislang eingesetzte, konventionelle antivirale Behandlungsstrategien konzentrieren sich ausschließlich auf Substanzen und Methoden, die das Ziel haben, spezifisch die Vermehrung eines Virus zu unterdrücken, um damit der Infektion Einhalt zu gebieten. Dieses kann durch verschiedene Wirkungsmechanismen erreicht werden. Ein geeigneter Angriffspunkt der Vermehrung des Virus ist das spezifische Virusreplikations-System oder eines seiner Komponenten. So kann mit Antimetaboliten, wie AZT (HIV, AIDS) oder Acyclovir (HSV, Herpes) der virale Replikationsapparat gehemmt werden. Andere Strategien beziehen sich auf viruskodierte Enzyme, die Funktionen in ganz bestimmten Replikationsphasen des Virus übernehmen. Dazu gehören z. B. die virale Protease zur Spaltung von Polypeptid-Translationsprodukten oder auch Wirkstoffe, die z. B. den Zusammenbau eines Virus verhindern oder die Freisetzung des Virusgenoms in der Zelle nach Infektionen unterbinden.

Die genannten Strategien haben gemeinsam, daß sie nach relativ kurzer Zeit Resistenzphänomene hervorrufen, so daß die behandelten Viren eine medikamentöse Therapie unterlaufen. Die Zeitspanne für das Auftreten von Resistenzphänomenen kann dabei für verschiedene Wirkstoffe und Viren stark variieren und Tage bis Jahre dauern.

M. Eigen ist es gelungen, die Eigenschaft der schnellen Anpassungsfähigkeit der Quasi-Spezies-Verteilung zu erklären (Eigen, M. (1971), Naturwissenschaften 58, 465 - 523). Unter einer Quasi-Spezies ist zu verstehen, daß das "Wildtyp"-Genom nicht aus einer definierten Sequenz besteht, die alle Viren einer Spezies besitzen, sondern aus einer stabilen Verteilung von Sequenzen, deren häufigste spezifische Sequenz der Sequenz des Wildtyps entspricht. Sie ist gleichbedeutend mit der Konsensus-Sequenz, die man erhält, wenn an jeder Position oder Sequenz das jeweils am häufigsten vorkommende Nukleotid eingesetzt wird (D. A. Steinhauer und J. J. Holland (1987), Ann. Rev. Microbiol. 41, 409 - 433). Die Mehrzahl der Sequenzen ist jedoch nicht mit der Wildtyp-Sequenz identisch. Dies bedeutet, daß das virale Genom zwar makroskopisch als Konsensus-Sequenz definiert und bestimmbar ist, mikroskopisch aber als ein Gemisch vieler Mutanten vorliegt, die in ständiger Konkurrenz zueinander stehen. Diese Konkurrenz hält die genetische Information des Virus in einem dynamischen Gleichgewicht von Mutation und Selektion. Durch Selektion wird die durch Mutation modifizierte genetische Information immer so wiederhergestellt, daß die Infektionskette aufrechterhalten bleibt (Domingo, E., Sabo, D., Taniguchi, T. und Weissmann, L. (1978), Cell 13, 735 - 744).

Mit einer Quasi-Spezies-Verteilung ist es einer Population von Mikroorganismen möglich, sich veränderten Umweltbedingungen außerordentlich schnell anzupassen. Ändern sich zum Beispiel die Temperaturbedingungen in der Umgebung einer Viruspopulation, so ist es möglich, daß der bestehende Wildtyp keinesfalls die Variante ist, die sich am erfolgreichsten unter den neuen Bedingungen vermehrt. Eine andere Variante aus dem großen Spektrum der Quasi-Spezies-Verteilung ist eventuell viel besser geeignet, bei der geänderten Temperatur zu replizieren. Es bedarf möglicherweise auch nur weniger zusätzlicher Mutationen zu einer besser angepaßten Variante, wenn von einer Variante der Quasi-Spezies ausgegangen wird anstatt von der Wildtyp-Sequenz. Durch diesen Mechanismus wird die große Flexibilität verständlich, mit der sich Mikroorganismen veränderten Umweltbedingungen in erstaunlich kurzen Zeiträumen anzupassen vermögen.

Diese Flexibilität kann mit einem hohen Anteil letaler oder partiell defekter Nachkommen in der Viruspopulation bezahlt werden. Tatsächlich ist in normalen Viruspopulationen meistens nur ein Bruchteil der freigesetzten Viren infektiös. Eine Erhöhung der Fehleinbaurate des viralen Replikationssystems kann die virale Population z. B. dann nicht verkraften, wenn die Fehleinbaurate über die theoretische Fehlerschwelle, die das Replikationssystem besitzt, hinweggehoben wird. Der Anteil an defekten Nachkommen wird dann so hoch, daß die Infektionskette durch Viren, die noch infektiös sind, auf Dauer nicht mehr aufrechterhalten werden kann.

Diese Strategie ist aus zwei Gründen, insbesondere spezifisch gegen eine Ausbreitung von Viren, vor allem RNA-Viren, gerichtet. In den meisten Fällen enthalten Viren - im Gegensatz zu ihren Wirten - RNA als genetisches Material. Um eine Vermehrung der Viren zu ermöglichen, muß das Virus wenigstens die wichtigsten Teile des Replikationsapparates selbst kodieren, da es nicht auf Bestandteile des Wirts zurückgreifen kann. Durch das Fehlen von komplizierten Fehlerkorrekturmechanismen sind der Replikationsgenauigkeit durch die physikalisch/chemische Beschaffenheit der Basenpaare enge Grenzen gesetzt. Bei RNA-Viren werden Fehleinbauraten im Bereich von 10⁻³ bis 10⁻⁵ realisiert. Desweiteren ist auch die Selektion bei viralen Genomen eingeschränkt. Wirtszellen teilen sich nach Verdoppelung ihrer Genome und fehlerhafte Chromosomen machen sich durch Nachteile für die Zelle sofort bemerkbar, sofern funktional benötigte Genorte betroffen sind. Virusgenome häufen sich dagegen in infizierten Zellen zu großen Populationen an. Defekte Genprodukte auf Einzelgenomen bewirken keine Nachteile für das fehlerhafte Genom selbst, denn alle Genome partizipieren in gleicher Weise an der gemeinsam Produktion. Auch bei der Replikation gibt es keine Benachteiligung von fehlerhaften Matrizen, so daß der Fehlerfortpflanzung zunächst praktisch kein Hindernis entgegensteht. Nur bei einigen wenigen Viren werden in der späten Infektionsphase die infektiösen Stränge von einer Matrize (Master Template) im Sinne eines Rolling-Circle-Modells abgelesen und somit die Fehlerfortpflanzung eingedämmt. Defekte im Genom eines Virus werden erst bei der nächsten Infektion manifest und können erst dann durch Selektion ausgemerzt werden.

Theoretische Berechnungen unterstützen den Befund, daß eine Verteilung von Quasi-Spezies-Informationen nur solange über beliebige Zeiträume stabil bleibt, solange eine bestimmte Fehlerschwelle des Replikationsapparates nicht überschritten wird. Computersimulationen haben eindeutige Korrelationen zwischen einem Muster von Selektionsvorteilen, einer Replikationsfehlerrate und der Stabilität der Quasi-Spezies-Verteilung ergeben. Wird diese Fehlerschwelle überschritten, so sagt die Berechnung ein Zerfließen der Information und somit das Ende der stabilen Quasi-Spezies-Verteilung in der Nachbarschaft der Wildtyp-Sequenz voraus.

Die von Manfred Eigen beschriebenen Zusammenhänge verdeutlichen das Dilemma in der die klassische Wirkstoffsuche durch Screening der aktiven Stoffe steckt. Werden antivirale Stoffe entdeckt, welche die Ausbreitung der Viruspopulation durch Vermehrung (Replikation) verhindern, so wird durch diese Stoffe ein "Selektionsdruck" auf die virale Quasi-Spezies-Verteilung ausgeübt, so daß sich resistente Mutanten bilden, die die Grundlage für eine neue virale Quasi-Spezies-Verteilung bilden. Sicherlich werden einige dieser Quasi-Spezies wiederum infektiös sein, so daß die virale Infektion durch eine neue Quasi-Spezies-Verteilung aufrecht erhalten wird. Die heutigen gängigen Konzepte zum sogenannten "Drug-Screening" sind mithin sogar gefährlich, weil immer die Gefahr besteht, daß durch diesen ungewollten Selektionsdruck, neue möglicherweise sogar stärker pathogene Viren induziert werden.

Aufgabe der vorliegenden Erfindung ist es, ein allgemein anwendbares Verfahren bereitzustellen, durch das Viruspopulationen bestimmte pathogene Eigenschaften verlieren, insbesondere die Eigenschaft der Infektiosität. Damit verbunden ist die Schaffung eines Mittels, mit dem virale Infektionen therapeutisch und prophylaktisch behandelt werden können, ohne die Bildung resistenter Viruspopulationen zu fördern. Das der Erfindung zu Grunde liegende technische Problem wird durch ein Verfahren gemäß den Merkmalen des Anspruchs 1 gelöst; die Unteransprüche 2 bis 15 betreffen bevorzugte Ausführungsformen des erfindungsgemäßen Verfahrens.

Die in den Ansprüchen 16 bis 18 beschriebenen Nukleinsäurensind vorzugsweise geeignet, im erfindungsgemäßen Verfahren die Instabilisierung von Quasi-Spezies-Verteilungen durch Verringerung eines Überlegenheitsparameters (s) zu bewirken.

Zur Durchführung des erfindungsgemäßen Verfahrens ist insbesondere ein Mittel nach einem der Ansprüche 19 bis 23 geeignet.

Die theoretisch abgeleitete Fehlerschwellenbeziehung (siehe unten) beinhaltet die Parameter s Überlegenheitsparameter (Superiorität) und (1-q) (Fehleinbaurate). Diese Parameter bilden die Basis des erfindungsgemäßen Verfahrens zur Überschreitung der maximal zulässigen Fehlerrate, der theoretischen Fehlerschwelle. Auf diese Weise wird die Quasi-Spezies-Verteilung instabilisiert.

Das erfindungsgemäße Verfahren nutzt die Instabilisierung einer viralen Quasi-Spezies-Verteilung aus, die auf der Replikation dieser Verteilung mit einem fehlerhaften Replikationssystem beruht. Dabei weist das fehlerhafte Replikationssystem eine Fehleinbaurate oberhalb der Fehleinbaurate des viralen Wildtyp-Replikationssystem auf. Die Fehleinbaurate stellt ein Maß für die Genauigkeit dar, mit der ein gegebenes virales Replikationssystem einer Quasi-Spezies-Verteilung ein Genom zu replizieren vermag.

Die Fehleinbaurate der Polymerase aus HIV-1 beträgt zum Beispiel 1 x 10⁻⁴. Dies bedeutet, daß größenordnungsmäßig nicht mehr als 10 kb-Genome stabil amplifiziert werden können. Dies entspricht in etwa der Länge des natürlichen Virus-Genoms.

Eigen et al. (1971) leiten aus zunächst rein theoretischen Überlegungen die Fehlerschwelle eines replikativen Systems ab. Es wird eine Beziehung aufgestellt zwischen den Größen q, m, s:${\text{m = ln s(1-q)}}^{\text{-1}}$
s: Für biologische Systeme grob vereinfachend wird der spezifischen Sequenz höchster Konzentration (Konsensus-Sequenz, ehemalige "Wildtyp"-Definition) ein Überlegenheitsfaktor (s) der Replikationseffizienz im Vergleich zur Replikationseffizienz der Gesamtheit aller potentiellen Mutanten zugeordnet.
m: Die Länge der zu replizierenden Sequenz in Nukleotiden oder Basenpaaren.
q: Die relative, durchschnittliche Rate für den korrekten Einbau des komplementären Watson-Crick Nukleotides, bezogen auf die einzelne Position. (1-q) entspricht der Fehlerrate.

Wenn es sich auch um eine Näherungsformel handelt, so beschreibt sie doch klar, daß ein replikatives System mit realen Kenngrößen q, m und s nur unterhalb einer bestimmten Fehlerschwelle replizieren kann, ohne daß die Information zerfließt.

Bisherige antivirale Strategien versuchen, die Replikation als solche zu unterdrücken. Das hier vorgestellte Verfahren basiert auf Erkenntnissen aus der Fehlerschwellenbeziehung. Über die Fehlerschwellenbeziehung ergeben sich drei Parameter, die erfindungsgemäß genutzt werden können, um eine Replikation jenseits der natürlichen Fehlerschwelle zu induzieren. Ein zentraler Parameter ist, wie weiter ausgeführt wird, der Parameter q, der durch die erfindungsgemäßen Maßnahmen auf geringere Werte zu drücken ist, ohne die Effizienz der Replikation insgesamt zu beeinflußen.

Der Parameter m ist nutzbar, wenn unter Beibehaltung der übrigen charakteristischen Daten die Länge der zu replizierenden Sequenz erhöht würde durch künstliche Verlängerung des Genoms. Dies geschieht mitunter in Unkenntnis der Konsequenz und unerwünscht dann, wenn z. B. bei der Herstellung rekombinanter Produkte deren kodierende Sequenz in filamentöse Phagen eingebaut wird und eine Instabilität der Phagen beobachtet wird. Hier wird offensichtlich die Fehlerschwellenbeziehung verletzt.

Wenn m = In s(1-q)⁻¹, dann ist die tatsächliche Fehleinbaurate des natürlichen Systems gleich der theoretischen Fehlerschwelle dieses Systems. Wird nun diese theoretische Fehlerschwelle überschritten, dann werden vermehrt Mutanten der Quasi-Spezies-Verteilung gebildet, was dann schließlich zu einer Verbreiterung der Quasi-Spezies-Verteilung führt.

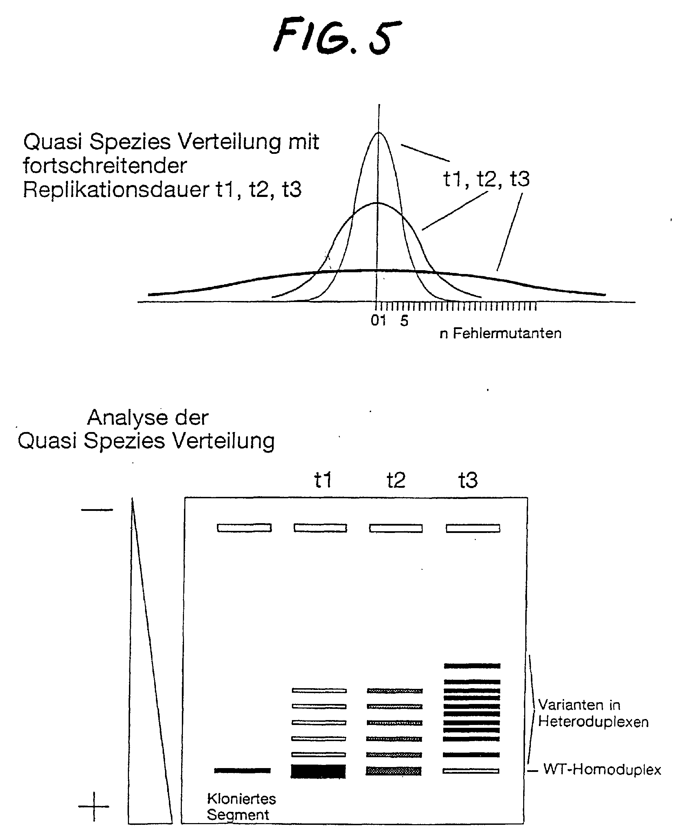

Dieser Zusammenhang ist in Fig. 5 skizziert. Die Figur 5 beschreibt schematisch die Quasi-Spezies-Verteilung einer Wildtyp-Population eines Virus. Die Abszisse gibt die Anzahl der Sequenzänderungen an, x=(0) bedeutet die Konsequenz des Virus, die als "Wildtyp" bezeichnet wird. Der zugehörige y-Wert zeigt ihren jeweiligen Anteil an der Population. Die Experimente von Domingo et al. (1978) am E. coli Virus Qß haben gezeigt, daß der Wildtyp lediglich die relativ häufigste, spezifische Sequenz darstellt. Die Mehrheit der Bakteriophagen stellt jedoch 1-, 2- und Mehr-Fehlermutanten dieser Sequenz dar, die in ihrer Gesamtheit eine Verteilung bildet, die die Wildtyp-Sequenz ergibt. Die Breite der Verteilung bleibt bei gleichbleibenden Wachstumsbedingungen normalerweise stabil. Das Mutantenspektrum erweitert sich jedoch, wenn die Replikation mit größeren Fehlern behaftet ist . Wenn die Replikation jenseits der erlaubten Fehlerschwelle vonstatten geht, erreicht die Quasi-Spezies-Verteilung kein neues Gleichgewicht. Die Verteilung wird mit fortschreitender Replikation (t1, t2, t3) kontinuierlich breiter, bis letztlich die Information vollständig zerfließt. Es kommt zur "Fehlerkatastrophe". Das Virus stirbt aus.

Ein wichtiges Kriterium des erfindungsgemäßen Verfahrens ist die Forderung, daß die Replikation der Quasi-Spezies-Verteilung, welche das Replikationssystem mit höherer Fehleinbaurate als der natürlichen Verteilung aufweist, mindestens so effizent verläuft wie die Replikation der Quasi-Spezies-Verteilung des viralen Wildtyps.

Eine zweite zentrale Voraussetzung des erfindungsgemäßen Verfahrens besteht darin, daß die Polymerase oder Polymeraseuntereinheit spezifisch für das jeweilige Virus die entsprechenden Viren jenseits der Fehlerschwelle repliziert, ohne dabei weniger effizient als die entsprechende Wildtypkomponente zu sein. Unter Effizienz ist dabei die Syntheserate der Genome der neuen Virionen zu verstehen. Es ist somit erforderlich, daß die Geschwindigkeitskonstanten für die Prozesse der Initiation, der Elongation und der Termination nicht beträchtlich von den jeweiligen Konstanten der Wildtypeinheiten abweichen, so daß insgesamt die genannte Bedingung zumindest erfüllt ist. Vorzugsweise bedeutet dies sogar einen erheblich beschleunigten Prozeß, der sich dann einstellen kann, wenn zum Beispiel Korrekturmechanismen ("proof reading") weniger oder gar nicht beansprucht werden.

Das erfindungsgemäße Verfahren beruht auf der überraschenden Erkenntnis, daß, im Sinne einer evolutiven Virusausbreitung, eine fehlerbedingte Degeneration bei hoher Amplifikationsrate für die Virusvermehrung ähnlich ungünstig ist, wie die Hemmung der Virusreplikation. Erfindungsgemäß ergeben sich noch weitere Aspekte der Erfindung zugrundeliegenden Strategie, die mit der Tendenz zusammenhängen, resistente Varianten zu erzeugen, die eine antivirale Therapie unterlaufen können.

Diese unerwünschten Phänomene werden gegenwärtig bei der AZT-Behandlung von HIV-infizierten Patienten offensichtlich. Während sich zunächst ein positiver Behandlungserfolg einzustellen scheint und fallende Virustiter beobachtet werden, stellen sich im Zeitraum weniger Wochen bis Monate AZT-resistente Virusvarianten ein. Eine Übertragung in in vitro Zellkulturen zeigt in diesen Fällen sehr deutlich, daß eine Hemmung der Virusreplikation mit diesen neuen Varianten eine um Größenordnungen höhere Dosierung des Therapeutikums notwendig machen würde, um lediglich eine halbmaximale Virusreplikation zu erreichen. Das verbietet sich jedoch aufgrund der systemischen Belastung des erythropoietischen Systems mit derart toxischen Konzentrationen an AZT.

Die fehlerhafte Replikation der viralen Nukleinsäure kann erfindungsgemäß vorzugsweise durch Einwirkung einer chemischen Substanz induziert werden. Dabei kann die Substanz als Antimetabolit oder allosterischer Effektor des Replikationssystems wirken. Die Substanz ist vorzugsweise so beschaffen, daß sie nicht mit den zellulären Enzymsystemen in Wechselwirkung tritt, um gegebenenfalls toxische Nebenwirkungen zu verhindern. Nebenwirkungen solcher Art treten fast zwingend in Erscheinung, wenn als Mechanismus zur Eindämmung einer Virusinfektion der Eingriff in die Replikation als Hemmung des Replikationssystems benutzt wird. Als Beispiel hierfür sei auf die negative Auswirkung von AZT auf das erythropoietische System bei der Behandlung von HIV Infektionen hingewiesen.

In einer bevorzugten Ausführungsform des erfindungsgemäßen Verfahrens stellt das fehlerhafte Replikationssystem eine Variante aus einem natürlichen Mutantenspektrum der Quasi-Spezies dar. Das fehlerhafte Replikationssystem kann auch eine durch Mutagenese erzeugte Mutante sein.

Vorzugsweise werden die Zielzellen der Virusinfektion (Zielzellen) durch Einschleusen eines viralen Replikationssystems in die Viruspopulation mit nachfolgender Infektion der Zielzellen in die Lage versetzt, ein Virus oberhalb der Replikations-Fehlerschwelle des viralen Replikationssystems zu replizieren. Dies bedeutet, daß die Replikationsfehlerrate dann höher liegt als diejenige der betreffenden Wildtyp Quasi-Spezies-Verteilung. Auch durch direktes Einschleusen eines viralen Replikationssystems oder Komponenten eines viralen Replikationssystem in die Zielzellen, können letztere in die Lage versetzt werden, ein Virus oberhalb der Replikations-Fehlerschwelle des viralen Replikationssystems, d. h. mit höherer Replikationsfehlerrate als derjenigen der betreffenden stabilen Quasi-Spezies-Verteilung, zu replizieren. Essentiell ist dabei aber immer die Bedingung, daß die Effizienz der Replikation der Mutante mit dem fehlerhaften Replikationssystem mindestens so gut ist wie diejenige der entsprechenden Wildtyp Quasi-Spezies-Verteilung.

Als bevorzugte Replikationssysteme werden RNA oder DNA Polymerasen oder Cofaktoren von RNA oder DNA Polymerasen eingesetzt.

Erfindungsgemäß wird vorzugsweise die Einschleusung des fehlerhaften Replikationssystems in die Viruspopulation durch Transformation von Individuen der betreffenden Viruspopulation oder der Zielzelle in an sich bekannter Weise durchgeführt. Dabei kommen die bekannten Verfahren der Gentherapie in Betracht.

Die Einschleusung des fehlerhaften Replikationssystems kann aber auch durch Superinfektion der Zielzelle mit Defektviren der gleichen Spezies erfolgen, welche das fehlerhafte Replikationssystem tragen.

Bei Viren bedeutet die Replikation oberhalb der natürlichen Fehlerschwelle der stabilen Quasi-Spezies-Verteilung fast immer auch eine Replikation oberhalb der zulässigen Fehlerschwelle. Dabei darf die Effizienz der Replikation nicht schlechter sein als bei der Quasi-Spezies-Verteilung des Wildtyps, da immer auch die korrekt arbeitenden Polymerasen zugegen sein können. Wird diese Bedingung nicht eingehalten und die Mutanten mit fehlerhaftem Replikationssystem nicht so effizient vermehrt, werden letztere nach mehreren Replikationszyklen derartig ausgedünnt sein, daß sie in der Quasi-Spezies-Verteilung keine Rolle mehr spielen.

In der Alternative 1 b) gemäß dem Hauptanspruch 1 werden vorzugsweise Nukleinsäuresequenzen eingesetzt, die erhältlich sind durch Reaktion von Nukleotiden und einem viralen Replikationssystem sowie anderer zur Vermehrung von Viren notwendigen Faktoren wie Puffersalze und Energieträger. Dabei werden von dem viralen Replikationssystem aus den Nukleotiden Oligo- oder Polynukleotide gebildet, deren ausschließliche genetische Information vorzugsweise lediglich die eigene maximale Amplifikation beinhaltet. Dieses System, vorzugsweise in zellfreier Form, optimiert durch Selektion also Oligo- oder Polynukleotide auf maximale Amplifikation dieser Sequenz durch Vermittlung des viralen Replikationssystems. Dabei erhält man unter anderen Bedingungen, beispielsweise Einsatz anderer viraler Replikationssysteme, entsprechende verschiedene Oligo- oder Polynukleotidsequenzen. Ebenfalls kann durch Einwirkung äußerer Einflüsse wie Temperatur oder Pufferbedingungen eine andere Oligo- oder Polynukleotidsequenz induziert werden.

Die erfindungsgemäß einsetzbare Nukleinsäuresequenz (Replikatoren und Replikatorvorläufer) sind vorzugsweise Nukleinsäuresequenzen, die teilweise homolog oder identisch sind mit solchen Sequenzen, die in vitro oder intrazellulär entstehen, wenn durch oder unter Mitwirkung des viralen Replikationssystems konkurrierend auf die am schnellsten replizierende Variante selektioniert wird, ohne daß alle oder einige Funktionen erhalten bleiben, die für das Wildtyp-Virus erforderlich sind, insbesondere proteinkodierende Funktionen oder die Expression regulierende Funktionen. Dies bedeutet praktisch, daß Nukleinsäuresequenzen eingesetzt werden, die außer der Information besonders gut vermehrt werden zu können, keine weiteren viralen genetischen Informationen mehr aufweisen müssen.

In den vergangenen Jahren sind zwar unterschiedliche antivirale Strategien diskutiert worden, die RNA-Moleküle verwenden, die mit viraler RNA in Wechselwirkung treten.

So ist Antisense-RNA bekannt, die über Hybridisierung an virale RNA direkt die Translation viraler Proteine verhindert (Coleman et al. 1984, Coleman et al. 1985, Hirashima et al. 1986) oder sie auf diese Weise für zelleigene RNase-Aktivität besonders exponiert (Agrawal et al., 1988). Es muß aber davon ausgegangen werden, daß die hierfür benötigte intrazelluläre Bildung von Doppelstrang-RNA-Strukturen zwischen Antisense-RNA und Sense-RNA unter nativen Bedingungen kein effizienter Prozeß ist. Desweiteren sind Ribozyme, die über Antisense-Bereiche spezifisch virale RNA-Sequenzen erkennen und schneiden können bekannt (Sarver et al., 1990; Rossi et al., 1990; Weizsäcker, 1992). Solche Ribozyme können aber auch eine hohe systemische Belastung darstellen, aufgrund ihrer Toxizität gegen zelluläre RNA. Desweiteren sind seit den ersten Versuchen von Spiegelman mit dem Qβ-System innerhalb dieses Systems Moleküle in Evolutionsexperimenten auf maximale Replikationsfähigkeit unter verschiedenen Randbedingungen optimiert worden (Mills et al., 1987, Levisohn et al., 1989, Biebricher, 1987, Bauer, 1990). Kleine RNA-Replikatoren sind aber auch für andere virale Polymerasen bekannt geworden (Konarska et al, 1989, Leary et al., 1991).

Virale Polymerasen können effektiv an der Replikation des natürlichen Substrates (virales Genom) gehindert werden, wenn ein Replikator angeboten wird, der mindestens eine der folgenden Eigenschaften aufweist:
- der Replikator hat intrazellulär eine signifikant kürzere Replikationszeit als das natürliche virale Substrat;
- die Bindungskonstante eines Replikators an die zugehörige Polymerase ist größer als die der natürlichen Nukleinsäure;
- der Replikator besitzt vorzugsweise möglichst wenige, besonders bevorzugt keine funktionalen Bindestellen für Effektoren mit negativem Rückkoppelungseffekt auf die Replikation, wie zum Beispiel ribosomale Bindestellen oder Bindestellen für inhibierende Faktoren aus einer späten Phase der Replikation wie z. B. Coat-Proteine.

Die erfindungsgemäße Strategie hat den Vorteil, daß die nicht infizierte Wirtszelle den Replikator oder das Replikator-Vorläufermolekül nur in geringer Menge exprimiert und erst nach Infektion in Gegenwart der viralen Replikase für eine starke Amplifikation sorgt. Diese Strategie läßt somit den Wirkstoff autokatalytisch spezifisch in der infizierten Zelle entstehen und stellt somit eine geradezu ideale Form des "Drug Targeting" dar. Abgesehen von der ohnehin als gering einzuschätzenden systemischen Toxizität einer kleinen für den Organismus funktionslosen Nukleinsäure wie RNA oder DNA in geringer Konzentration werden durch diesen Mechanismus mögliche Nebenwirkungen weiter eingeschränkt.

In einer bevorzugten Ausführungsform eines transgenen Wirtssystems kann auch die Möglichkeit realisiert werden, den Replikator oder vorzugsweise seinen Vorläufer überhaupt nicht konstitutiv zu exprimieren, sondern die Nukleinsäure unter die Kontrolle eines Regulationselementes zu stellen. Als solches Regulationselement kommt insbesondere ein Promotor oder ein Replikationsursprung, der in trans von viralen Komponenten nach Infektionen aktiviert wird und den Replikator oder seinen Vorläufer freisetzt.

Beim Einsatz von RNA-Replikatoren, deren Vorläufermoleküle als DNA in die Wirtszelle eingeschleust werden, kann vorzugsweise das zelluläre posttranskriptionelle Reifungssystem, wie im unten angegebenen Beispiel beschrieben, eingesetzt werden, um authentische Endstücke der Replikator-RNA zu erzeugen. Es ist ansonsten nicht ohne weiteres möglich, Transkriptionsprodukte zu erzeugen, die den essentiellen Replikase-Bindungseigenschaften der Enden von Replikatormolekülen entsprechen. Das unten angegebene Beispiel beschreibt den Vorläufer einer RNA mit tRNA-Fusion, die über das zelluläre System der RNase P prozessiert wird.

Die erfindungsgemäß als Replikator oder Replikatorvorläufer einsetzbare Nukleinsäuresequenz trägt einen Nukleinsäureanteil aus dem mit Hilfe von Polymerasen intrazellulär der Replikator entsteht, wobei das Vorläufermolekül zusätzliche Anteile enthält, die den Transport in eine Zielzelle ermöglicht und/oder die eine stabile Integration des Replikatorsegmentes in das Genom der Zielzelle ermöglichen.

Das folgende Beispiel beschreibt die Herstellung eines Replikatorvorläufers und des Replikators zur Verhinderung der Ausbreitung des Qβ-Virus in E. coli.

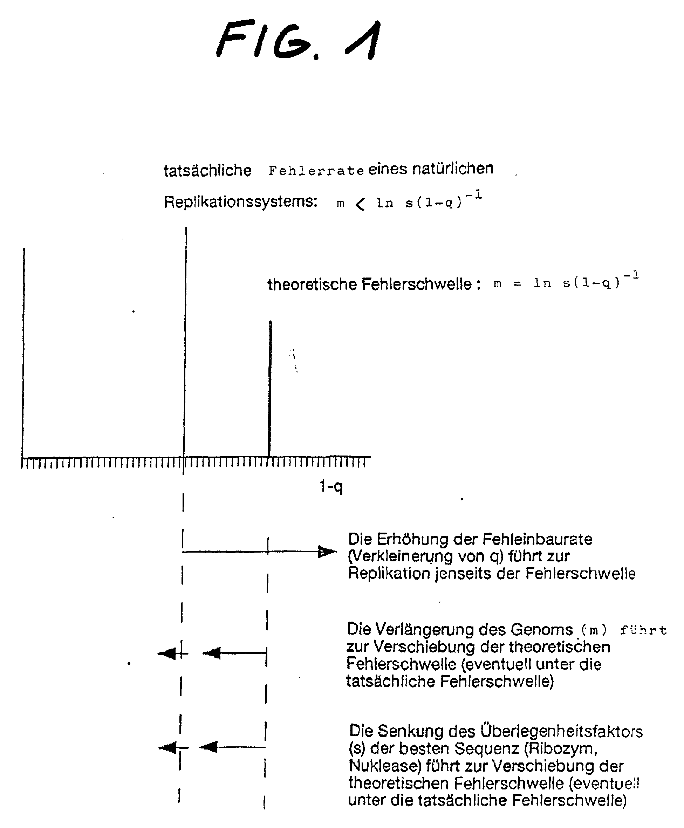

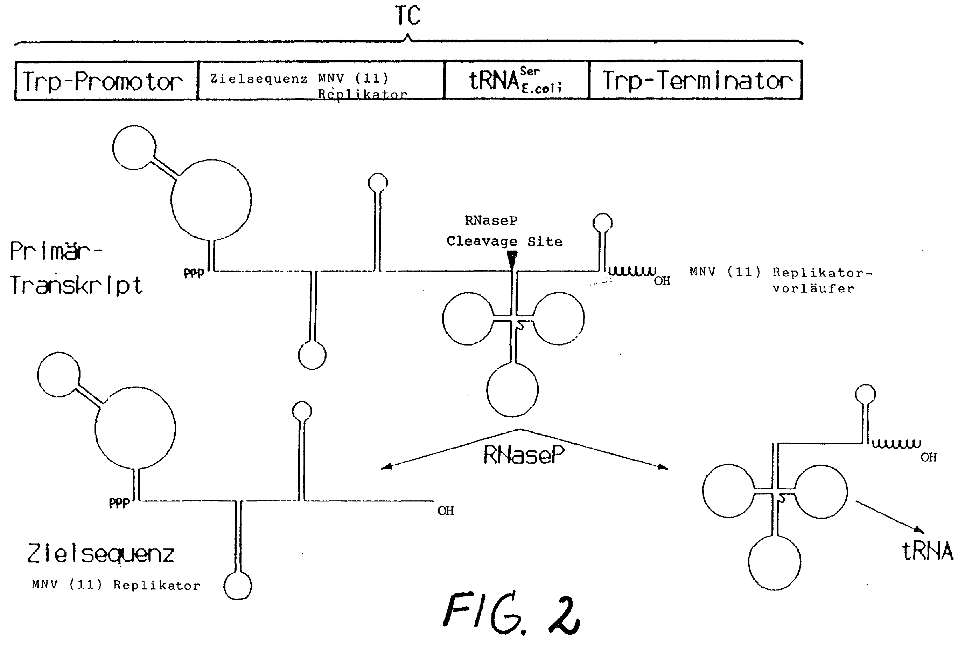

Figur 2 beschreibt die Konstruktion des Vorläufer-Transkriptes mit Replikatoreigenschaft zur Inhibierung der Qß-Amplifikationen in E. coli. Das Primärtranskript steht unter der Kontrolle eines Trp-Promotors. Die eigentliche funktionsfähige Zielsequenz erfordert ein präzises 3'-Ende, um als Replikator für die Qβ-Replikase zu wirken. Dies ist durch den unmittelbaren Einsatz eines direkten Transkriptes, das zum Beispiel ein Terminationssignal erfordert nur schlecht oder gar nicht zu erreichen. Es läßt sich jedoch durch den Einbau einer t-RNA-Fusionssequenz das zelluläre RNase P-System ausnutzen, daß die Primärsequenz in den funktionalen Replikator prozessiert.

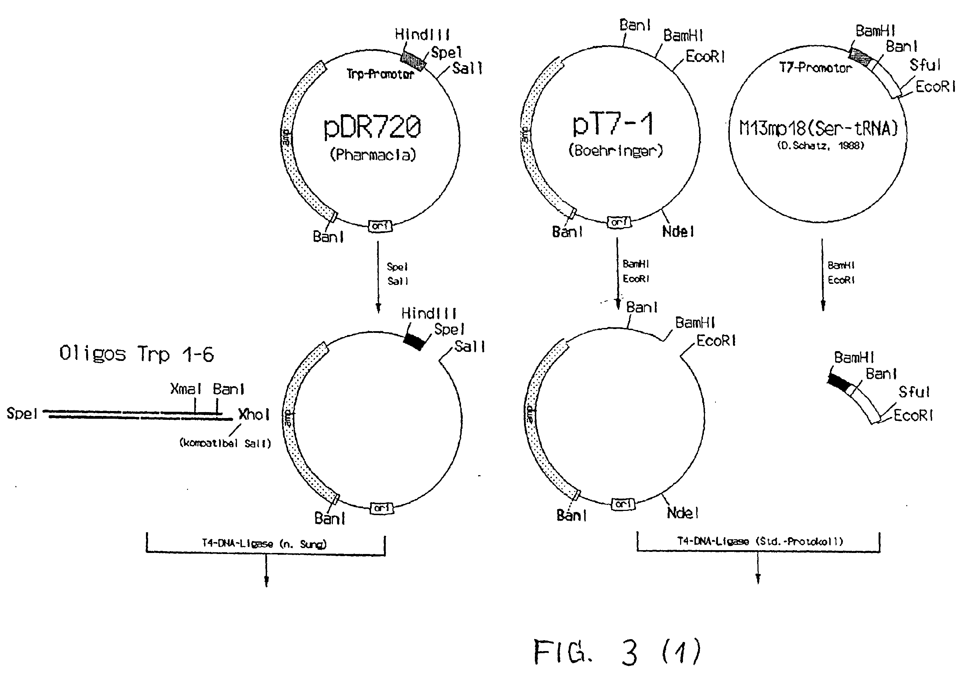

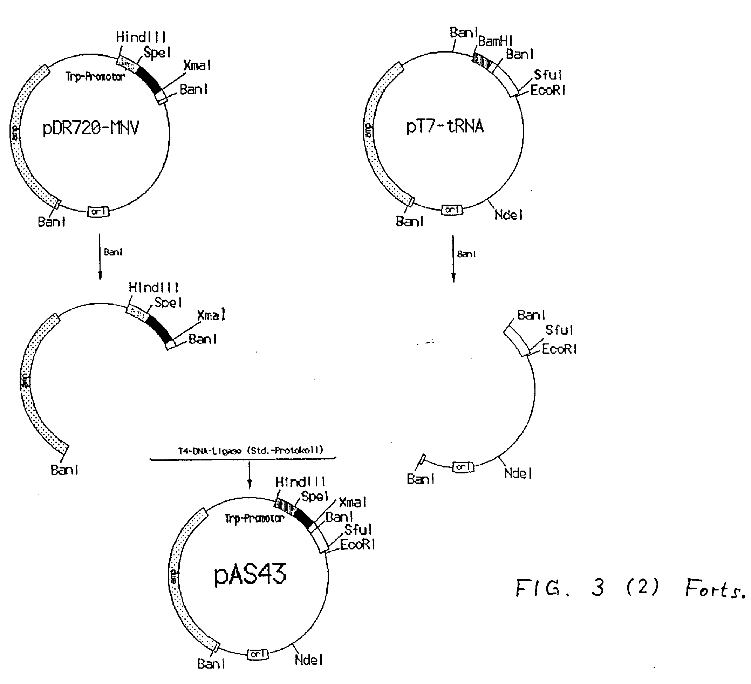

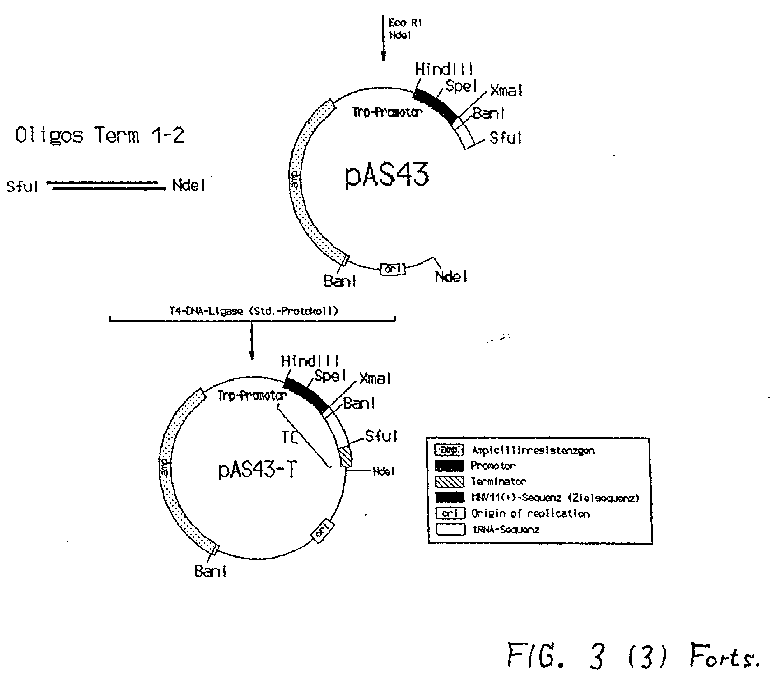

Die Figur 3 beschreibt die Konstruktion des in Figur 2 beschriebenen Konstrukts, welches in ein Plasmid integriert wurde (Pas 43t). Die dort angewendeten Verfahren sind an sich bekannte Verfahren der Gentechnik.

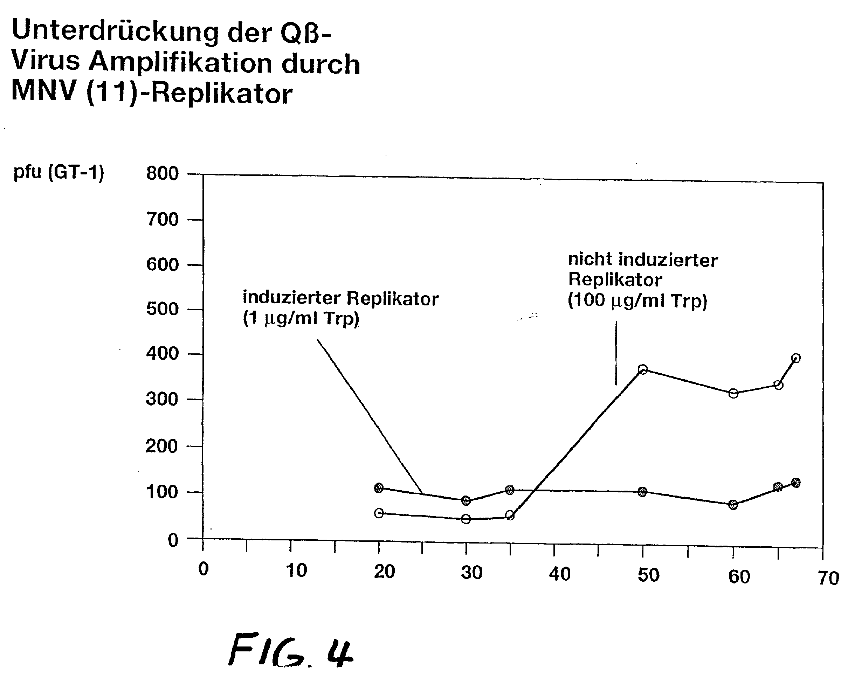

Die Figur 4 zeigt die blockierende Wirkung der eingeschleusten Nukleinsäuresequenz auf die Qβ-Replikation. Die Experimente wurden in Minimalmedium in einem Zell-Stat-System durchgeführt. Es ist ersichtlich, daß es nur bei abgeschalteten Trp-Promotor (100 µg/ml) zu einem "Burst" der Zellen und einer initialen Freisetzung von Qß-Viren nach ca. 50 Minuten kommt.

In einer bevorzugten Variante wird der charakteristische Überlegenheitsparameter (s) der Virusreplikation durch eine Kombination des Replikationssystems mit einer oder mehrerer Nukleasen, Ribozymen und/oder Antisense-RNA verkleinert. Dabei ist mindestens eine Nuklease und/oder ein Ribozym und/oder Antisense-RNA gegen Bestandteile des jeweiligen Virus-Genoms gerichtet. Die Fehlerschwelle, d. h. die noch maximal tolerierbare Fehleinbaurate des entsprechenden Replikationssystems wird sowohl durch die virale Genomlänge, als auch durch die Überlegenheit der am erfolgreichsten replizierenden Sequenz definiert. Wird jedoch die erfolgreichste replizierende Sequenz durch Nukleasen und/oder Ribozyme zerstört, führt dies konsequenterweise zu einer Verringerung des charakteristischen Überlegenheitsparameters (s) .

Zusammenfassend läßt sich also sagen, daß sich erfindungsgemäß die Instabilisierung einer viralen Quasi-Spezies durch die Alternative 1 b) des Hauptanspruchs erreichen läßt, indem Replikatoren, also Nukleinsäuresequenzen mit Hilfe des viralen Replikationssystems mit der viralen Nukleinsäure koamplifiziert werden. Dabei muß die Effizienz der Replikation der Replikatoren höher sein als diejenige der viralen Konsensus-Sequenz, deren Superiorität damit nicht mehr gegeben ist.

Erfindungsgemäß wird das virale Gen für das Replikationssystem mit der höheren Fehleinbaurate gemäß Alternative a) des erfindungsgemäßen Verfahrens mit weiteren regulatorischen Genabschnitten versehen, die im transformierten Virusindividum oder der transformierten Zielzelle weitere Steuerungsfunktionen übernehmen. Sie können zum Beispiel dafür Sorge tragen, daß das fehlerhafte Expressionssystem, insbesondere nach erfolgter Virusinfektion aktiviert wird. Vorzugsweise sind trans-aktivierbare Regulationssequenzen, die als regulatorische Genabschnitte eingesetzt werden können, geeignet, auch für eine erwünschte höhere Replikationsrate der Viruspopulation zu sorgen.

Die Zielzellen der viralen Infektion bzw. die Wirtszellen der Viren können einzellige Organismen oder Bakterien sein, vor allem aber pflanzliche Zellen oder tierische Wirtszellen wie Blutzellen oder erythropoietische Stammzellen.

Die vorliegende Erfindung basiert auf der Erkenntnis, daß die Fehleinbaurate der an der Replikation beteiligten Enzyme direkt mit der Länge des zu replizierenden Genoms verknüpft ist. Das Genom ist dabei ein Maß für die Komplexität der genetischen Information und dem Replikationsvorteil der Wildtyp-Sequenz gegenüber den Sequenzen des Mutantenspektrums. Eine Analyse der Polymerasefehleinbauraten natürlicher Organismen und Viren hat ergeben, daß Viren mit großer Genomlänge über Polymerasen mit geringer Fehleinbaurate verfügen, wohingegen kurzkettige Genome durchaus mit großer Fehleinbaurate zuverlässig repliziert werden können, ohne daß die gespeicherte Information zerfließt. Die gemessenen in vitro-Daten stimmen gut mit den theoretisch vorhergesagten Daten überein, wenn man unterstellt, daß ein virales System seine evolutiven Möglichkeiten dann optimal ausnutzt, wenn seine Genomlänge dicht unterhalb der kritischen Länge bezogen auf die Fehlerschwelle der Replikation (wie oben ausgeführt) liegt.

Erfindungsgemäß wird nun die Korrelation zwischen Fehlerschwelle und der viralen Replikation und der Genomlänge verletzt. Dies bedeutet, daß die Fehlerschwelle bei der Replikation überschritten werden muß, so daß die genetische Information des betreffenden infektiösen Virus zerfließt. Das geschieht um so besser, je stärker das betreffende Virusindividuum vermehrt wird. Dieses Konzept läuft allen bekannten Bestrebungen und Konzepten entgegen, virale Infektionen mittels Arzneimitteln einzudämmen. Gerade bei der Behandlung von HIV ist man z. B. eher bemüht, den Mechanismus zu unterdrücken, mit dem es dem Virus offensichtlich gelingt, allen Versuchen des Immunsystems zu entkommen, die Virusvermehrung und -ausbreitung durch neutralisierende Antikörper und zelluläre Abwehrmechanismen zu unterdrücken. So ist literaturbekannt, daß HIV-Varianten in der immundominaten Region V3 des gp120 immer wieder einen Ausweg von neutralisierenden Antikörpern ermöglichen. Deshalb sind viele therapeutische Anstrengungen darauf ausgerichtet, das Entstehen von Varianten, d. h. das Entstehen von Mutanten, die gerade durch die fehlerhafte Replikation entstehen und vermehrt werden zu unterbinden. Scheinbar führt die erfindungsgemäße Strategie also in die falsche Richtung, wenn darauf abgestellt wird, Viren in verstärktem Maße zu vermehren. Dies ist jedoch nicht der Fall, denn das Virus büßt drastisch an Infektiosität ein, sobald es mit Replikationssystemen jenseits der Fehlerschwelle vermehrt wird und gerade dann, wenn es besonders vehement vermehrt wird. Es ist bekannt, daß die Erzeugung virusresistenter Wirtsorganismen gelingt, wenn in ein wesentliches Regulationssystem der Virusreplikation eingegriffen wird. Virale Informationsträger werden nur bis zu einer bestimmten zahlenmäßigen Obergrenze in einer Wirtszelle vermehrt. Die Replikation unterliegt also einer viruskodierten Kontrolle. Häufig sind an dieser Replikationsbegrenzung virale Coat-Proteine beteiligt. Wenn die ersten entsprechenden Translationsprodukte in einer Zelle auftauchen, besitzen sie bestimmte Loci am Replikationsursprung oder an der Kontrollregion der Replikase oder Polymerase und führen nachfolgend zu einer Blockierung der Replikationsinitiation. Basis einer ganzen Reihe von Ansätzen zur Behandlung und Prophylaxe viraler Erkrankungen ist die konstitutive Expression der Coat-Proteine pathogener Viren über transgene Konstrukte. Dieser Ansatz wird insbesondere im Pflanzenschutz verfolgt, indem transgene Pflanzen diese viralen Coat-Proteine exprimieren. Die konstitutive Expression von Coat-Proteinen scheint bei mehreren Viren eine Resistenz gegenüber einer natürlichen Infektion zu bewirken. Die Tatsache jedoch, daß zum Beispiel bei einem Tabakmosaikvirus (TMV) die Resistenz aufgehoben ist, wenn mit nackter RNA infiziert wird, spricht dafür, daß sehr unterschiedliche Resistenzmechanismen die Wirkung der Coat-Proteine erklären könnten. Der viruskodierten Blockierung der Replikation an einer bestimmten zahlenmäßigen Obergrenze kommt aus der Sicht des Virus eine wichtige Bedeutung zu. Wenn innerhalb einer Wirtszelle Replikationskopien eines Virus selbst wieder die Matrize für eine Replikation innerhalb derselben Wirtszelle darstellen, so bedeutet dies, daß bereits fehlerhaft replizierte Genome die Matrize für weitere Replikationsrunden bilden, so daß bei der nächsten Replikationsrunde noch fehlerhaftere Kopien entstehen. Viren, die sich dementsprechend nach dem sogenannten "Rolling-Circle-Prinzip" replizieren, haben einen Ausweg aus diesem Dilemma gefunden, indem nur eine Kopie jeweils als Matrize fungiert.

Werden durch Verwendung von Replikationsprodukten als Matrize innerhalb einer Zelle mit vielen Fehlern behaftete Genome synthetisiert, ist dies zunächst ohne Konsequenzen, da keine Selektion der entstehenden Viren auf Infektiosität stattfindet. Es können nicht-funktionsfähige Gene oder auch Gene, die zu minderwertigen Produkten führen, wie zum Beispiel zu einer Replikase hoher Fehlerrate, in trans komplementiert werden. Es werden somit letztlich virale Genome "verpackt", die durch Replikasen hoher Fehleinbaurate jenseits der Fehlerschwelle repliziert werden. Dadurch wird die "gesunde" Population (die Quasi-Spezies-Verteilung des Wildtyps) der Viren verdünnt durch insuffiziente Viren, die eingeschränkt infektiös und eingeschränkt replikationsfähig sind. Dies ist nicht im Sinne einer stabilen Viruspopulation, wie sie für das Virus vorteilhaft ist.

Am Beispiel der Qβ-Phagen-Infektion in E. coli wird dieser Mechanismus näher erläutert. Nach Infektion des bakteriellen Wirtes werden ca. 1.000 Replikaseuntereinheiten pro Zelle synthetisiert, woraufhin die Translation des Replikase-Locus unterbrochen wird. Für den Translationsstop konnte das virale Coat-Protein identifiziert werden mit einer Bindungsstelle innerhalb der Replikase-Translationsregion.

Eine E.coli-Zelle hat nur eine begrenzte Kapazität, Phagen zu synthetisieren, so daß eine Überschußproduktion von viralen Genomen überflüssig ist. Von A. Schwienhorst und B. Lindemann wurde experimentell bestätigt, daß für das Virus fatale Folgen auftreten, wenn zu viele fehlerhafte Genomkopien und daraus folgend fehlerhafte Replikasekopien hergestellt werden. Bei der Replikation der + Qβ-RNA werden in einer Zelle nicht nur die Ursprungsmatrize eingesetzt, sondern auch deren Replikationsprodukte, die nach dem Konzept der Quasi-Spezies-Verteilung statistisch in ihrer Mehrzahl 1- bis 2-Fehlermutanten darstellen. Da diese Mutanten innerhalb eines Wirtes vielen Selektionskriterien nicht unterliegen, zum Beispiel ihre Infektiosität, häufen sich viele unvorteilhafte Virusvarianten an. Daher kann die Viruspopulation bestehend aus Quasi-Spezies-Verteilungen stabil überleben, die mit ca. 1.000 Replikationsmolekülen pro Zelle erzeugt werden.

Wenn jedoch der Coat-Protein vermittelte Regulationsmechanismus außer Kraft gesetzt wird, wird unmittelbar der oben beschriebene Effekt sowie die Problematik des Schutzes mit transgenen Coat-Protein-Produzenten als Zielzellen deutlich. Derart transformierte E.coli-Zellen wurden mit Qβ-Phagen infiziert. Zunächst konnte, wie vermutet, ein effektiver Schutz der Wirtszellen durch die Blockierung der Replikase-Translation beobachtet werden. Nach einiger Zeit wurden jedoch resistente Varianten beobachtet, die durch den Selektionsdruck induziert worden waren. Eine nähere Analyse ergab, daß sich unter diesen Selektionsbedingungen solche Virus-RNA-Mutanten durchsetzten, deren Bindungsstelle für das Hüllprotein defekt waren und sich durch Hüllprotein daher nicht mehr reprimieren ließen. Es ist anzunehmen, daß damit auch die Begrenzung der Replikasesynthese wegfällt.

Dadurch wird deutlich, daß dieser recht moderne Ansatz, nämlich transgene Coat-Proteine exprimierende Wirtszellen einzusetzen, auf Dauer auch resistente Virusmutanten hervorbringen wird. Hierzu wird es keiner langen Zeitspanne bedürfen, wenn, wie im Falle der Replikationsblockierung bei Qβ-Phagen, durch das Coat-Protein eine einzige Punktmutation allein die Resistenzbarriere durchbricht.

Die erfindungsgemäße Strategie hingegen, die im Sinne der Informationserhaltung zu stark mit Fehlern behaftete Mutanten hervorbringt, führt über den Zeitraum länger andauernder Infektion zu einer Degeneration der Quasi-Spezies-Verteilung, die dem Virus Wildtyp entspricht, und somit zu deren Aussterben. Erfindungsgemäß tritt dies ein, wenn die Fehleinbaurate des fehlerhaften Replikationssystems nur unwesentlich oberhalb der evolutiv optimierten Fehlerschwelle liegt. Erfindungsgemäß wird die Fehlerrate der Virusreplikation über die evolutiv zulässige Schwelle angehoben, wobei die Replikation nicht gehemmt werden darf, sondern mindestens so effizient verlaufen muß wie diejenige des Virus-Wildtyps. Dies kann durch mehrere Maßnahmen erfolgen.

So kann ein Wirkstoff, der mit einem Replikationsprozeß in einer Weise interferiert, daß es zu einer spezifischen Erhöhung der Fehleinbaurate des viralen Replikationssystems kommt, eingesetzt werden. Mit den bisherigen antiviralen Screeningsystemen kann ein solcher Wirkstoff nicht ermittelt werden. Der Einsatz eines Systems, wie es erfindungsgemäß beschrieben ist, in Kombination mit in vitro-Zellkulturen erlaubt aber ein effektives Screening auf derartige Mutanten einer Polymerase aus Proben von fehlerhaften Genompopulationen, wie sie für E.coli-Viren oben beschrieben sind.

Das Screening auf HIV wird zum Beispiel mit klonierten HIV-Varianten in infizierten Zielzellen wie peripheren Blutzellen oder transformierten Lymphozytenzellen wie HUT 78 durchgeführt. Das virusreplizierende System wird dabei mit potentiellen Wirkstoffen verschiedener Konzentration inkubiert. Ein repräsentativer Genlocus der Virus-RNA oder -DNA wird nach erfolgter Kultivierung sodann mit einer Polymerase geringer Fehlerrate in vitro amplifiziert. Das Amplifikationsprodukt wird mit einer entsprechenden klonierten Sonde der Ausgangsvarianten hybridisiert und zum Beispiel mit einem Detektionssystem wie in der PCT/EP 90/01366 beschrieben auf die Anwesenheit unkorrekter, in den Zellen amplifizierter, Virusvarianten untersucht. Auf diese Weise lassen sich solche Wirkstoffe entdecken, die die Fehlerschwelle überschreiten, aber nicht eine Replikation verhindern und nicht notwendigerweise die Infektiosität meßbar beeinflussen. Von solchen Wirkstoffen kann erwartet werden, daß sie auch alloster wirken können. Sie sollten somit nicht zwangsläufig auch mit zellulären Polymerasesystemen interagieren, im Gegensatz zu Konzepten, die auf dem Antimetabolitenprinzip beruhen, zum Beispiel Nukleotidanaloga wie AZT. Wenn die Replikation als solche nicht unmittelbar unterbunden wird, ist darüber hinaus auch keine direkte Rückkopplung zu erwarten, die einer therapieresistenten Punktmutante sofort zu einem vitalen Selektionsvorteil verhilft.

In einer besonders bevorzugten Ausführungsform läßt sich das erfindungsgemäße Verfahren anwenden, wenn transgene Systeme eingesetzt werden wie es für den pflanzlichen Bereich bei der Coat-Protein-Produktion bereits praktiziert wird. Stammzellen ohne differenzierte potentielle Empfängerzellen infizierter oder gefährdeter Patienten können mit einem Gen für eine Viruspolymerase (RNA-Replikase, reverse Transkriptase oder DNA-abhängige DNA-Polymerase) erhöhter Fehlerrate, die durch oben erwähnte Screening-Verfahren gewonnen werden können, transformiert werden. Welche Virusmutanten auch immer eine fehlerhafte Replikation hervorbringen, sie treffen auf eine Zielzelle, die bereits das virale Replikationssystem mit erhöhter Fehleinbaurate oder entscheidender Cofaktoren oder Untereinheiten davon bereitstellt. Alle Virusvarianten werden oberhalb ihrer charakteristischen Fehlerschwelle repliziert. Damit kann es keiner Virusvariante gelingen, Resistenzen zu entwickeln. Die Information wird mit fortschreitender Amplifikation zerfließen. Das Verfahren wird um so wirksamer sein, je weniger die Virusamplifikation als solche inhibiert wird.

Das Konzept des Schutzes von Wirtszellen gegen virale Infektionen durch Überschreitung der Fehlerschwelle der virusspezifischen Replikase/Polymerase unter Beibehaltung der Replikationseffizienz kann am Beispiel E.coli für das Qβ-Virus näher beschrieben werden, wenn man E.coli mit dem Replikasegen unter Kontrolle eines eigenen Promoters transformiert. Die Replikase entstammt dabei dem Mutantenspektrum von Qβ und muß eine erhöhte Fehleinbaurate gegenüber dem viralen Wildtyp aufweisen. Die Replikationseffizienz der Replikase darf sich jedoch in zellfreien in vitro-Systemen anhand zum Beispiel der Replikation von Midi- und Minivarianten nicht von derjenigen der Wildtyp-Replikase unterscheiden. Die Replikasemutante enthaltenden E.coli Zellen als Modellsystem für gentherapierte potentielle Wirtszellen lassen sich z. B. transient im "Zell-Staten" mit Wildtyp-Qβ-Phagen infizieren. Nach anfänglicher Infektion geht die Anzahl der Plaques pro ml im Vergleich zu nicht transformierten Zellen deutlich zurück. Die Infektion mit T-Phagen verläuft jedoch für beide Zelltypen ähnlich, wie es zu erwarten ist, da die Qβ-Replikase bei der Replikation von T-Phagen nicht interferiert.

Wenn Wirtsvarianten mit verlorener Resistenz gegen Qβ-Phagen-Infektion auftreten, ist das kein Effekt, der im Widerspruch zur vorliegenden Erfindung steht. Es handelt sich dann bei den analysierten Stämmen mit wiedererlangter Suszeptibilität um Stammvarianten mit defekter Mutantenreplikase oder mit einer Promoter-Variante, die durch fehlerhafte E.coli-Replikation entstanden sind. Diese Form der Resistenzbildung ist auch bei höheren Zellen theoretisch möglich, wenn auch aufgrund niedrigerer Zellzahlen und niedrigerer Teilungsraten und höherer Präzision der Replikation um Größenordnungen unwahrscheinlicher, so daß ihr in der Praxis keine Bedeutung zukommen wird.

Es stehen mehrere Verfahren unterschiedlicher Komplexität zur Verfügung, um aus einem natürlichen oder künstlichen Spektrum von Mutanten einer Polymerase eine geeignete herauszufinden. Für E.coli-Bakterien wurde bereits eine bevorzugte Quelle genannt, um aus dem Spektrum einer verbreiterten Quasi-Spezies-Verteilung Replikasen erhöhter Fehleinbaurate zu fischen.

Es gilt zu testen, ob eine unterschiedliche Morphologie der oben beschriebenen Qβ-Plaques auf eine fehlerhafte replizierende Polymerase (Replikase) zurückgeführt werden kann. Ein einfacher genetischer Test ist eine Plaque-Reinigung der morphologisch veränderten Plaques. Bleibt die morphologische Besonderheit mehr oder weniger einheitlich erhalten, so deutet dies nicht auf eine Mutation im Polymerasegenabschnitt hin, sondern in einem anderen Segment des polycistronischen Genoms. Wenn hingegen wiederum Plaques einer größeren morphologischen Vielfalt entstehen, ist dies ein starker Hinweis auf eine fehlerhaft arbeitende Polymerase. Die Größe der Plaques wiederum kann als Indiz für eine schnelle Synthesekinetik gewertet werden. Von großen Plaques wird erwartet, daß sie auch über eine schnelle Replikase verfügen. Somit kann der Aufwand zur molekularbiologisch charakterisierten Auffindung der gewünschten Replikase verkleinert werden. Mit der Technik der Temperaturgradienten-Gelelektrophorese läßt sich ein fraglicher Plaque sicher daraufhin analysieren, ob er tatsächlich Phagen mit einer Replikase größerer Fehleinbaurate enthält.

Die Temperaturgradienten-Gelelektrophorese kann hochsensitiv Mutationen wie Punktmutationen in einem bestimmten Nukleinsäuresegment nachweisen. Es lassen sich auf diese Weise auch Mutanten in einer Population von Sequenzen aufspüren. Der Nachweis von Mutanten wird gewöhnlich auf der Ebene der doppelsträngigen DNA durchgeführt. So läßt sich z. B. ein Mutantenspektrum in vitro amplifizierter Nukleinsäure-Segmente untersuchen, indem nach erfolgter Amplifikation in einem Denaturierungs-/ Renaturierungsschritt nahezu alle Mutanten in Heteroduplexe überführt werden (siehe Figur 5). Wenn eine spezifische Sequenz wie z. B. ein kloniertes Segment im Überschuß vorliegt, wird eine unterrepräsentierte Mutante nach Denaturierung nicht mehr ihren eigenen Gegenstrang finden. Vielmehr bildet sie Heteroduplexe mit anderen Gegensträngen, statistisch am häufigsten mit der am häufigsten vorkommenden Sequenz. Während die Homoduplexe auf einem parallelen Temperaturgradienten-Elektrophoresegel in einer Bande höchster Mobilität wandern, werden die verschiedenen Heteroduplexe entlang des Temperaturgradienten spezifisch verlangsamt, da sich die Moleküle partiell in Einzelstrangstrukturen entfalten. In einem Gemisch aus einem Überschuß einheitlicher Sequenzen mit Mutanten dieser Sequenz erhält man somit das typische Bild der Figur 5, Klone A - D. In der am schnellsten wandernden Bande wandern die Homoduplexe gefolgt von einer Bandenschar verschiedener Heteroduplexe (PCT/EP 90/01366).

Aus der Intensität der Mutantenbanden im Verhältnis zur Intensität der Homoduplexbande läßt sich auch auf die relative Häufigkeit der Mutanten schließen bzw. auf die Fehleinbaurate des replizierenden Enzymsystems (W. Thilly). Analog läßt sich im erfindungsgemäßen Sinne eine Population spezifischer Sequenzen testen, die unter Mitwirkung der gesuchten fehlerhaften Polymerase entstanden sind. Diese Fragmente lassen sich im Temperaturgradienten-Gelelektrophorese-System entweder direkt testen oder nach Amplifikation wie durch die PCR-Reaktion mit oder ohne vorherige Umschreibung in DNA durch eine Reverse-Transkriptase. Um die Fehlerrate einer Replikase abzuschätzen, kann dem Gemisch vorzugsweise ein radioaktiv markiertes Segment zugeführt werden, dessen Sequenz mit derjenigen des Homoduplex identisch ist. Es lassen sich somit nach erfolgter Denaturierung und Renaturierung (Figur 5) die Radioaktivitäten der Homoduplex-Bande und die der Heteroduplexe durch Zählen bestimmen. Das Verhältnis der gemessenen Aktivitäten ist dann ein direktes Maß für die Fehlerhäufigkeit des replizierenden Systems bzw. der Replikase. Vorraussetzung ist bei Einführung eines enzymatischen Verstärkungsschrittes, daß die Fehlerhäufigkeit des Verstärkungsschrittes klein ist gegenüber der Fehlerhäufigkeit der vorangegangenen Replikation.

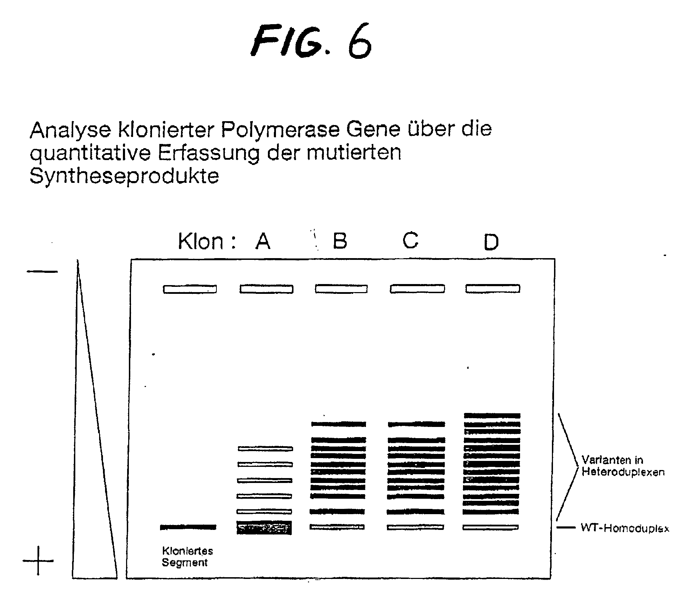

Mit diesem Verfahren läßt sich auch die schematisch in Figur 6 beschriebene Verbreiterung der Quasi-Spezies-Verteilung mit fortschreitender Infektionsdauer mit Replikation unter maßgeblicher Beteiligung der fehlerhaften Polymerase verfolgen. Während bei der Infektion einer nicht transformierten Zellpopulation mit einem Wildtyp-Virus die Mutantenverteilung ohne wechselnden Selektionsstress annähernd konstant bleibt, tritt unter Beteiligung des erfindungsgemäßen, fehlerhaften Replikationsprozesses eine Verbreiterung der Quasi-Spezies-Verteilung ein. In der Temperaturgradienten-Gelelektrophorese-Analyse zeigt sich dies in einem größer werdenden Verhältnis der Bandenintensität des Mutantenspektrums im Verhältnis zur Bande mit der größten relativen Häufigkeit, ehemals als Wildtyp bezeichnet.

Das Temperaturgradienten-Gelelektrophorese-System kann gleichfalls dazu herangezogen werden, um die Effizienz, d. h. die Replikationsgeschwindigkeit im oben beschriebenen Sinne zu messen, bzw. mit der des wt-Enzyms zu vergleichen. Wie von Henco et al. beschrieben (Nucleic Acids Res. (1990)), läßt sich das Temperaturgradienten-Gelelektrophorese-System zur genauen Quantifizierung von Nukleinsäuren in einer Probe verwenden, wobei die Genauigkeit ± 15% beträgt. Somit läßt sich die relative Syntheseleistung einer Wildtyp-Polymerase und einer Mutantenpolymerase im zellfreien in vitro-System oder in Zellkultur anhand ihrer jeweiligen Nukleinsäure-Syntheseprodukte quantifizieren.

Die beschriebenen Verfahren lassen sich analog zur Auffindung und Klassifizierung von Polymerasemutanten anderer Virus/Wirtsysteme heranziehen, die erfindungsgemäß einsetzbar sind. Wenn sich wie im Falle von Tabakmosaikvirus (TMV) analog zur Plaquebildung bei Bakterien klonale, virusverursachte Läsionen in Blättern nachweisen lassen, gibt es die Möglichkeit aus der Morphologie einzelner Läsionen auf die Anwesenheit von viralen Polymerasemutanten erhöhter Fehleinbaurate zu schließen. TMV verursacht auf N-Gen-Tabak (N. glutinosa, N. tabacum cv. Xanthi-nc bzw. Samsun NN) nekrotische Lokalläsionen (Atabekov). Diese Reaktion führt zur Lokalisierung der Virusinfektion um die Orte des primären Befalls. Temperatursensitive Mutanten sind bei erhöhter Temperatur (z. B. 32°C) nicht in der Lage, das entsprechende Produkt in funktionellem Zustand zu bilden. Es gibt solche Mutanten sowohl für das Hüllprotein als auch für das Transport-Protein. Unter nichtpermissiven Bedingungen sind die Funktionen der RNA-Umhüllung bzw. des Zell-zu-Zell-Transports gestört. Auf der anderen Seite ist die nekrotische Reaktion des N-Gen-Tabaks ebenfalls temperaturabhängig. Bei erhöhter Temperatur (z. B. 32°C) bleibt die Lokalisierung aus und das TMV (Wildtyp) breitet sich über die Blattspreite aus. Bei Rückführung auf Normaltemperatur kommt es zum Kollabieren des Gewebes. Die Nekrotisierung führt zu großflächigen pergamentartigen Abschnitten. Bei kombinierter Verwendung von N-Gen-Tabak und Ts-Mutanten ist ein anderes Bild zu erwarten. Obwohl die Nekrotisierung und Lokalisierung bei erhöhter Temperatur ausbleibt, kommt es zu keiner Virusausbreitung, weil die Transportfunktion unter nichtpermissiven Bedingungen ausgeschaltet ist. Bei Rückführung auf Normaltemperatur kommt es zur Nekrosebildung, als hätte es die Temperaturbehandlung nicht gegeben. Da es sich bei den experimentellen Mutanten des Virus um gut charakterisierte Punktmutanten handelt, kann anhand des Symptombildes nach differentieller Temperaturbehandlung die Rate der Rückmutation durch fehlerhafte Replikaseaktivität eingeschätzt werden.

## Patentansprüche

1. Verfahren zur Instabilisierung von viralen Quasi-Spezies-Verteilungen unter Vermeidung von Resistenzphänomenen durch Replikation der Nukleinsäuren der in der Quasi-Spezies-Verteilung vorhandenen Viren mittels eines fehlerhaften Replikationssystems,
- wobei das fehlerhafte Replikationssystem eine Fehleinbaurate für Nukleotide oberhalb der Fehleinbaurate des viralen Wild-Typ-Replikationssystem aufweist und
- wobei die Viren von dem Replikationssystem mit höherer Fehleinbaurate mindestens so effektiv repliziert werden wie es das Replikationssystem des Virus-Wildtyps leistet.

2. Verfahren nach Anspruch 1 mit einer negativen Beeinflussung der Replikation der Konsensus-Sequenz (Nukleinsäuresequenz des Virus-Wildtyps) im Verhältnis zu anderen replizierbaren Nukleinsäuren.

3. Verfahren gemäss einem der Ansprüche 1 und 2, wobei die fehlerhafte Replikation der viralen Nukleinsäure durch Einwirkung einer chemischen Substanz induziert wird.

4. Verfahren gemäss Anspruch 3, wobei die chemische Substanz als Antimetabolit oder allosterischer Effektor des Replikationssystems wirkt.

5. Verfahren gemäss einem der Ansprüche 1 bis 4, wobei das fehlerhafte Replikationssystem eine Variante aus einem natürlichen Mutantenspektrum der Quasi-Spezies darstellt oder durch Mutagenese erzeugte Mutante ist.

6. Verfahren nach einem der Ansprüche 1 bis 4, wobei über das Einschleusen eines viralen Replikationssystems in die Viruspopulation mit nachfolgender Infektion der Zielzellen der Virusinfektion (Zielzellen) oder durch direktes Einschleusen eines viralen Replikationssystems oder Komponenten eines viralen Replikationssystem in die Zielzellen, letztere in die Lage versetzt werden, einen infizierenden Wildtyp-Virus oberhalb der Replikations-Fehlerschwelle des viralen Replikationssystems, d.h. mit höherer Replikationsfehlerrate als derjenigen der betreffenden stabilen Quasi-Spezles-Verteilung, zu replizieren bei mindestens gleicher Effizienz der Replikation.

7. Verfahren nach einem der Ansprüche 1 bis 6, wobei die Replikationssysteme RNA oder DNA Polymerasen oder Cofaktoren von RNA oder DNA Polymerasen sind.

8. Verfahren nach einem der Ansprüche 1 bis 7, wobei die Einschleusung des fehlerhaften Replikationssystems in die Viruspopulation durch Transformation von Individuen der betreffenden Viruspopulation oder der Zielzelle in an sich bekannter Weise nach Verfahren der Gentherapie erfolgt.

9. Verfahren nach einem der Ansprüche 1 bis 7, wobei die Einschleusung des fehlerhaften Replikationssystems durch Superinfektion der Zielzelle mit Defektviren der gleichen Spezies erfolgt, die das fehlerhafte Replikationssystem tragen.

10. Verfahren nach einem der Ansprüche 1 bis 9, wobei das das virale Replikationssystem mit der höheren Replikationsfehlerrate tragende Gen durch klonale Selektion erhalten oder synthetisch hergestellt wurde und in ein Virusindividuum oder die Zielzelle mit an sich bekannten gentechnischen Verfahren eingeschleust wurde.

11. Verfahren nach mindestens einem der Ansprüche 1 bis 10, wobei das für das viralen Replikationssystem mit der höheren Fehlerrate kodierende Gen mit weiteren regulatorischen Genabschnitten versehen ist, die im transformierten Virusindividuum oder der transformierten Zielzelle weitere Steuerungsfunktionen übernehmen.

12. Verfahren nach Anspruch 11, wobei der weitere regulatorische Genabschnitt für eine höhere Vermehrungsrate der Viruspopulation Sorge trägt.

13. Verfahren nach Anspruch 2, wobei die andere replizierbare Nukleinsäure effizienter als die Nukleinsäure der Konsensus-Sequenz repliziert wird.

14. Verfahren nach einem der Ansprüche 1, 2 und/oder 13, wobei der charakteristische Überlegenheitsparameter (s) durch eine Kombination des Replikationssystems mit einer oder mehrerer Nukleasen und/oder Ribozymen und/oder Antisense-RNA verkleinert wird, wobei eine oder mehrere Nukleasen und/oder Ribozyme und/oder Antisense-RNA gegen Bestandteile des jeweiligen Virusgenoms gerichtet sind und/oder die andere replizierbare Nukleinsäure als Replikator oder Replikatorvorläufer in der nicht infizierten Zielzelle nur in geringer Konzentration vorliegt und erst nach Infektion durch die Polymerase des infizierenden Virus repliziert wird.

15. Verfahren zur Behandlung oder Prophylaxe von viralen Erkrankungen, wobei entweder die befallenen Zielzellen mit einem Vektorsystem, insbesondere einem viralen Vektorsystem, mit mindestens einem viralen Replikationssystem, das zu einem Replikationssystem mit höherer Fehleinbaurate führt transformiert sind oder die Zielzellen durch Einschleusen eines viralen Replikationssystems, das zu einer höheren Fehleinbauratefehlerrate führt, transformiert wurde oder die Zielzellen durch eine oder mehrere Substanzen behandelt werden, die eine erhöhte Fehleinbaurate der Replikation bewirken.

16. Verfahren nach einem der Ansprüche 1 bis 15, wobei die Wirtszellen als Zielzellen der viralen Infektion einzellige Organismen oder Bakterien, pflanzliche Zellen oder tierische Wirtszellen wie Blutzellen oder erythropoietische Stammzellen sind.

17. Mittel zur Durchführung des Verfahrens nach einem der Ansprüche 1 bis 16, enthaltend eine Nukleinsäure oder eine Nukleinsäure kodierend für eine Nukleinsäure, erhältlich durch Reaktion von Nukleotiden und einem viralen Replikationssystem sowie anderen zur Vermehrung von Viren notwendigen Faktoren unter Bildung von Oligo- oder Polynukleotiden, wobei ausschließlich auf maximale Amplifikation oder Oligo- oder Polynukleotide durch das virale Replikationssystems selektoniert wird.

18. Mittel nach Anspruch 17, enthaltend mindestens einen Genabschnitt kodierend für ein virales Replikationssystem und/oder einen Cofaktor eines viralen Replikationssystems, wobei das zu kodierende System zu einem viralen Replikationssystem mit einer höheren Fehleinbaurate, als durch das native Replikationssystem festgelegt, führt, wobei die Effizienz der Replikation zumindest erhalten bleibt.

19. Mittel nach Anspruch 17 und/oder 18, enthaltend mit dem Replikationssystem, das zu höheren Fehleinbauraten führt, transformierte Viren, Phagen oder eu- oder prokaryontischen Zellen und/oder entsprechend präparierte Phagen oder Plasmide zur Transformation der Zielzellen oder transformierte Zielzellen selbst.

20. Mittel nach einem der Ansprüche 17 bis 19, **dadurch gekennzeichnet, dass** sie als sogenannte Replikationsenzyme eine Replikation oberhalb der Eigen-Fehlerschwelle bewirken, bei mindestens gleicher Replikationseffizienz verglichen mit dem Wildtyp.
